# EUROPEAN PATENT APPLICATION

(11) **EP 4 484 424 A1**
(43) Date of publication of application: **01.01.2025**
(21) Application number: 23759204.3
(22) Date of filing: 22.02.2023
(51) Int. Cl.: C07D 413/12, C07D 267/10, A61K 31/553, A61P 11/00

(54) **DIPEPTIDYL PEPTIDASE 1 INHIBITOR POLYMORPH, PREPARATION METHOD AND USE THEREFOR**

(30) Priority: 22.02.2022 CN 202210160848
(71) Applicant: Haisco Pharmaceuticals Pte. Ltd., Singapore 079903 (SG)
(72) Inventor: FAN, Jiang, Chengdu, Sichuan 611130 (CN); DOU, Ying, Chengdu, Sichuan 611130 (CN); GONG, Zheng, Chengdu, Sichuan 611130 (CN); ZHU, Fengfei, Chengdu, Sichuan 611130 (CN)
(74) Representative: IP TRUST SERVICES
(86) International application number: PCT/CN2023/077649
(87) International publication number: WO 2023/160579

(57) **Abstract**

Disclosed in the present invention are a polymorph of a compound as shown in formula I, and a preparation method and use therefor. The polymorph of the present invention comprises a crystal form B, a crystal form C, and a crystal form D. The polymorph has excellent characteristics of high purity, good solubility, stable physical and chemical properties, resistance to high temperature, high humidity, and strong illumination, low hygroscopicity, etc.

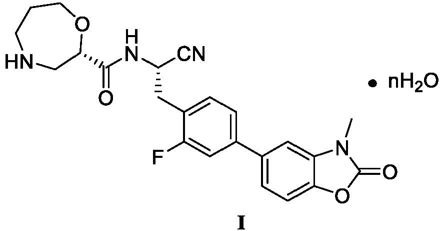

## Description

### Technical Field

The present invention belongs to the pharmaceutical field, and in particular relates to a dipeptidyl peptidase 1 inhibitor polymorph, and a preparation method therefor and the use thereof.

### Background Art

Dipeptidyl peptidase 1 (DPP1), also known as cathepsin C, is a cysteinyl protease of the lysosomal papain family involved in intracellular protein degradation. During neutrophil maturation, DPP1 activates neutrophil serine proteases (NSPs), including neutrophil elastase (NE), proteinase 3 (Pr3), and cathepsin G (CatG), by cleaving the N-terminal dipeptide of a target protein. DPP1 has been implicated in a variety of inflammatory diseases, including Wegener's granulomatosis, rheumatoid arthritis, lung inflammation and virus infection. Studies have shown that the inhibition of DPP1 can have a good therapeutic effect on highly inflammatory lung diseases caused by neutrophils, such as bronchiectasis, chronic obstructive pulmonary disease (COPD) and acute lung injury. Therefore, inhibiting the over-activation of NSPs by targeting DPP1 may have a potential therapeutic effect on bronchiectasis.

In patent application PCT/CN2020/114500, a DPP1 small molecule inhibitor as shown in the formula below is prepared. The compound exhibits relatively high DPP1 inhibitory activity and excellent bioavailability and pharmacokinetic characteristics and has the advantages of low toxicity and high safety. It is intended to be used to treat lung diseases such as non-cystic fibrosis bronchiectasis, chronic obstructive pulmonary disease (COPD), acute lung injury and cystic fibrosis bronchiectasis. Thus, developing a crystal form of a compound that is more suitable for drug formulation, especially a crystal form with improved stability, hygroscopicity, preservability and/or efficacy, so as to achieve good results in the pharmaceutical industry, has become an urgent problem to be solved.

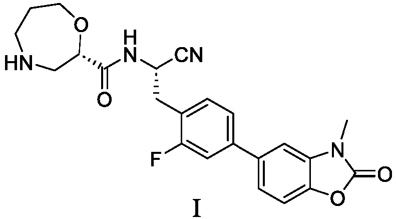

### Summary of the Invention

The present invention provides a polymorph of a compound as shown in formula I with a high suitability as a pharmaceutical. The polymorph has excellent characteristics of high purity, good solubility, stable physical and chemical properties, resistance to high temperature, high humidity, and strong illumination, low hygroscopicity, etc.

A polymorph of a compound as shown in formula I, 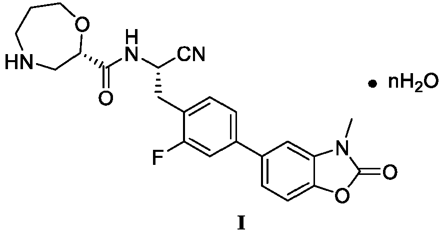 wherein n is 0 or 1.

In some embodiments, the polymorph of the compound as shown in formula I is an anhydrous crystal form, i.e., n is 0, and the polymorph is described as crystal form B, which has an X-ray powder diffraction pattern comprising characteristic diffraction peaks at 12.32 ± 0.2°, 14.30 ± 0.2°, 15.43 ± 0.2°, 16.38 ± 0.2° and 18.56 ± 0.2° 2θ, as determined by using Cu-Kα radiation.

Further, in some embodiments, the crystal form B has an X-ray powder diffraction pattern further comprising characteristic diffraction peaks at 17.06 ± 0.2°, 18.21 ± 0.2°, 18.94 ± 0.2°, 19.59 ± 0.2°, 20.79 ± 0.2°, 21.19 ± 0.2°, 22.55 ± 0.2°, 22.97 ± 0.2°, 23.37 ± 0.2°, 24.25 ± 0.2° and 25.46 ± 0.2° 2θ.

Further, in some embodiments, the crystal form B has an X-ray powder diffraction pattern comprising characteristic diffraction peaks at the following positions (2θ):

| No. | Pos. [°2θ] |
|---|---|
| 1 | 4.68 |
| 2 | 8.97 |
| 3 | 12.32 |
| 4 | 14.30 |
| 5 | 15.43 |
| 6 | 16.38 |
| 7 | 17.06 |
| 8 | 18.21 |
| 9 | 18.56 |
| 10 | 18.94 |
| 11 | 19.59 |
| 12 | 20.79 |
| 13 | 21.19 |
| 14 | 22.55 |
| 15 | 22.97 |
| 16 | 23.37 |
| 17 | 24.25 |
| 18 | 25.46 |
| 19 | 31.03 |

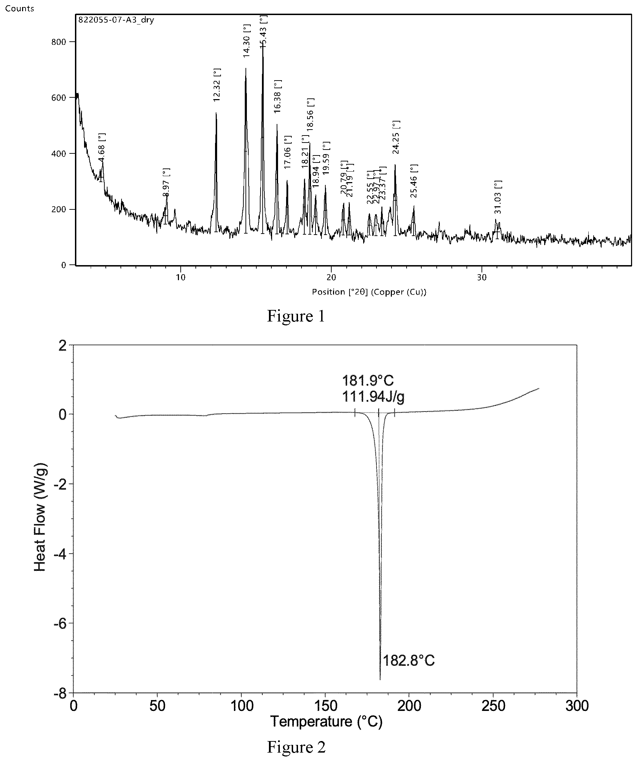

Further, in some embodiments, the crystal form B has an X-ray powder diffraction pattern substantially as shown in Figure 1.

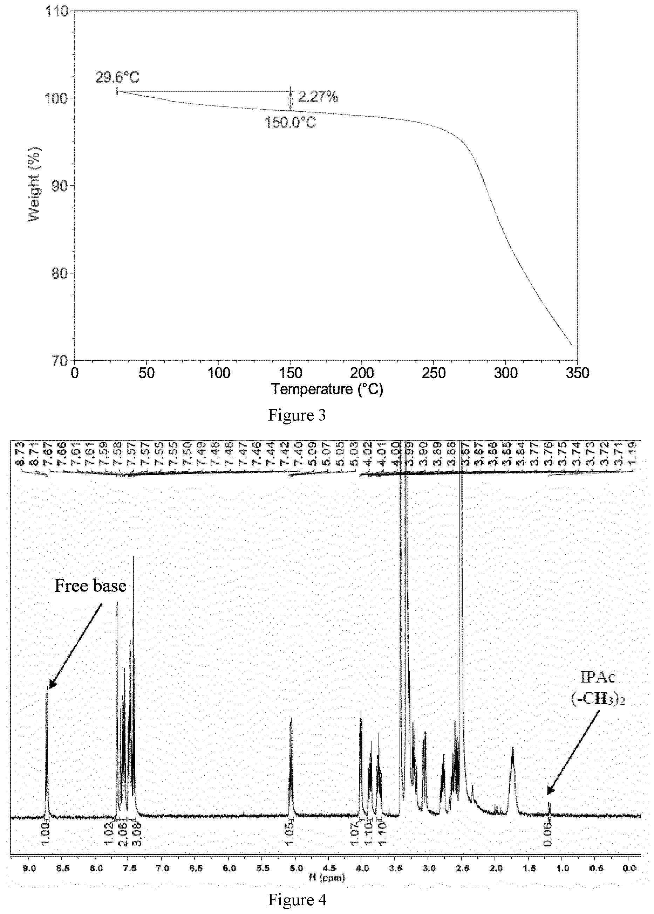

Further, in some embodiments, the crystal form B has a DSC pattern as shown in Figure 2 and/or a TGA pattern as shown in Figure 3.

In some embodiments, the polymorph of the compound as shown in formula I is a monohydrate crystal form, i.e., n is 1, and the polymorph is described as crystal form C, which has an X-ray powder diffraction pattern comprising characteristic diffraction peaks at 8.93 ± 0.2°, 12.10 ± 0.2°, 14.14 ± 0.2°, 20.38 ± 0.2°, 23.86 ± 0.2° and 25.07 ± 0.2° 20, as determined by using Cu-Kα radiation.

Further, in some embodiments, the crystal form C has an X-ray powder diffraction pattern further comprising characteristic diffraction peaks at 15.19 ± 0.2°, 15.92 ± 0.2°, 16.06 ± 0.2°, 17.55 ± 0.2°, 17.90 ± 0.2°, 18.90 ± 0.2°, 19.10 ± 0.2°, 20.81 ± 0.2° and 24.33 ± 0.2° 2θ.

Further, in some embodiments, the crystal form C has an X-ray powder diffraction pattern comprising characteristic diffraction peaks at the following positions (2θ):

| No. | Pos. [°2θ] |
|---|---|
| 1 | 4.68 |
| 2 | 8.93 |
| 3 | 12.10 |
| 4 | 14.14 |
| 5 | 15.19 |
| 6 | 15.92 |
| 7 | 16.06 |
| 8 | 17.55 |
| 9 | 17.90 |
| 10 | 18.90 |
| 11 | 19.10 |
| 12 | 20.38 |
| 13 | 20.81 |
| 14 | 23.32 |
| 15 | 23.86 |
| 16 | 24.33 |
| 17 | 25.07 |
| 18 | 25.91 |
| 19 | 26.99 |
| 20 | 28.45 |
| 21 | 29.67 |
| 22 | 31.41 |
| 23 | 36.26 |

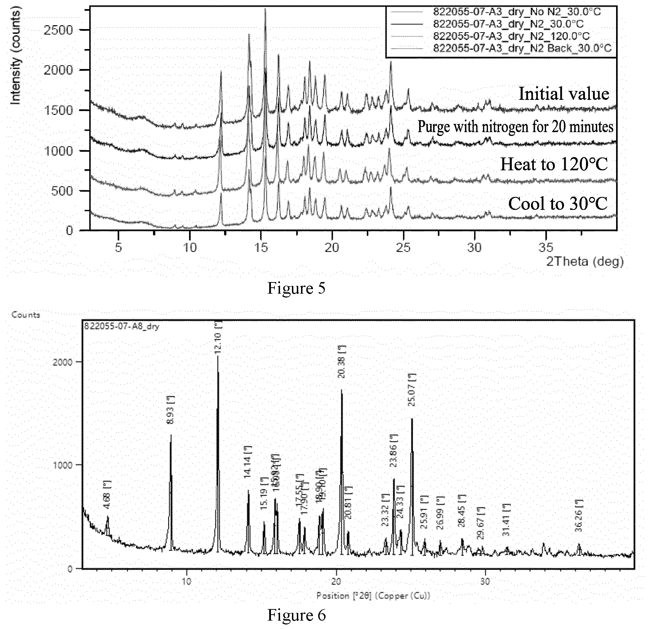

Further, in some embodiments, the crystal form C has an X-ray powder diffraction pattern substantially as shown in Figure 6.

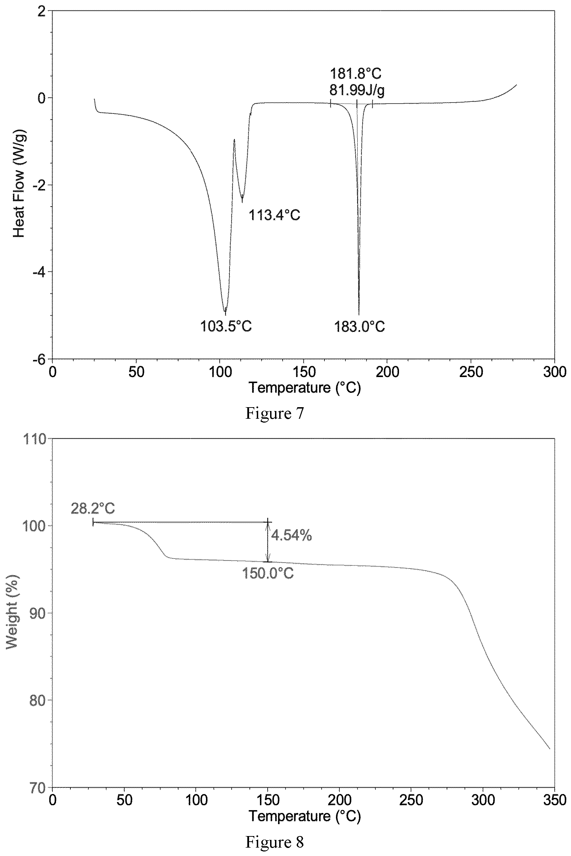

Further, in some embodiments, the crystal form C has a DSC pattern as shown in Figure 7 and/or a TGA pattern as shown in Figure 8.

In some embodiments, the polymorph of the compound as shown in formula I is an anhydrous crystal form, i.e., n is 0, and the polymorph is described as crystal form D, which has an X-ray powder diffraction pattern comprising characteristic diffraction peaks at 8.68 ± 0.2°, 11.74 ± 0.2°, 13.64 ± 0.2°, 17.40 ± 0.2°, 18.23 ± 0.2°, 20.12 ± 0.2° and 26.21 ± 0.2° 2θ, as determined by using Cu-Kα radiation.

Further, in some embodiments, the crystal form D has an X-ray powder diffraction pattern further comprising characteristic diffraction peaks at 8.99 ± 0.2°, 13.51 ± 0.2°, 27.49 ± 0.2°, 32.19 ± 0.2° and 32.97 ± 0.2° 2θ.

Further, in some embodiments, the crystal form D has an X-ray powder diffraction pattern comprising characteristic diffraction peaks at the following positions (2θ):

| No. | Pos. [°2θ] |
|---|---|
| 1 | 4.49 |
| 2 | 8.68 |
| 3 | 8.99 |
| 4 | 11.74 |
| 5 | 13.51 |
| 6 | 13.64 |
| 7 | 14.80 |
| 8 | 15.59 |
| 9 | 17.40 |
| 10 | 18.23 |
| 11 | 20.12 |
| 12 | 22.82 |
| 13 | 23.60 |
| 14 | 24.08 |
| 15 | 26.21 |
| 16 | 27.49 |
| 17 | 28.57 |
| 18 | 32.19 |
| 19 | 32.97 |
| 20 | 36.98 |

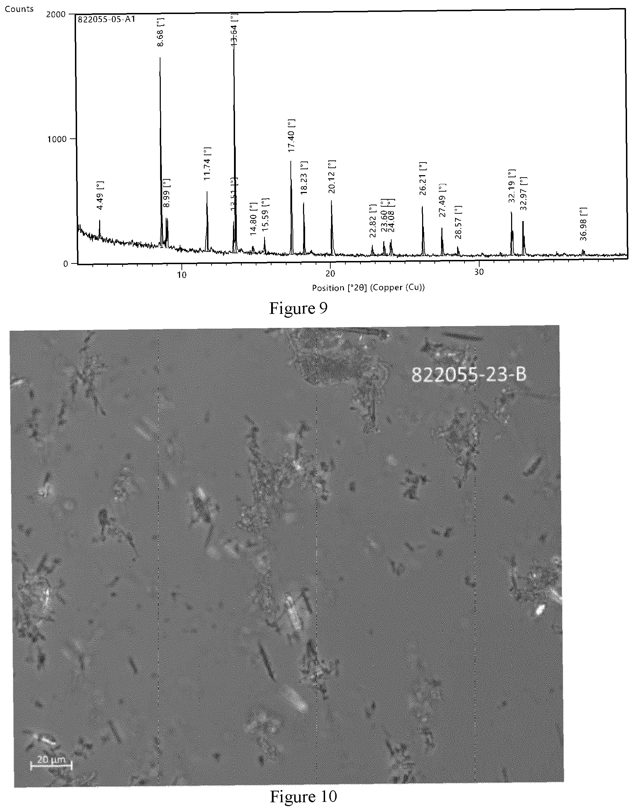

Further, in some embodiments, the crystal form D has an X-ray powder diffraction pattern substantially as shown in Figure 9.

Further, in some embodiments, with regard to the crystal form B, crystal form C or crystal form D of the present invention, the crystal has a particle size less than 100 µm; in some embodiments, the crystal has a particle size less than 90 µm; in some embodiments, the crystal has a particle size less than 80 µm; in some embodiments, the crystal has a particle size less than 70 µm; in some embodiments, the crystal has a particle size less than 60 µm; in some embodiments, the crystal has a particle size less than 50 µm; in some embodiments, the crystal has a particle size less than 40 µm; in some embodiments, the crystal has a particle size less than 30 µm; and in some embodiments, the crystal has a particle size less than 20 µm.

The present invention also provides a pharmaceutical composition, comprising a therapeutically effective amount of the crystal form B, crystal form C or crystal form D, and a pharmaceutically acceptable carrier and/or excipient.

The present invention also provides the use of the crystal form B, crystal form C or crystal form D or the pharmaceutical composition comprising same in the preparation of a medicament for treating a DPP1-mediated disease.

Further, the DPP1-mediated disease includes, but is not limited to non-cystic fibrosis bronchiectasis, cystic fibrosis bronchiectasis, acute lung injury, obstructive airway disease, bronchiectasis, cystic fibrosis, asthma, emphysema and chronic obstructive pulmonary disease.

The crystal form B, crystal form C or crystal form D of the present invention is present in an amount accounting for about 5 wt% to about 100 wt% of the bulk drug; in certain embodiments, in an amount accounting for about 10 wt% to about 100 wt% of the bulk drug; in certain embodiments, in an amount accounting for about 15 wt% to about 100 wt% of the bulk drug; in certain embodiments, in an amount accounting for about 20 wt% to about 100 wt% of the bulk drug; in certain embodiments, in an amount accounting for about 25 wt% to about 100 wt% of the bulk drug; in certain embodiments, in an amount accounting for about 30 wt% to about 100 wt% of the bulk drug; in certain embodiments, in an amount accounting for about 35 wt% to about 100 wt% of the bulk drug; in certain embodiments, in an amount accounting for about 40 wt% to about 100 wt% of the bulk drug; in certain embodiments, in an amount accounting for about 45 wt% to about 100 wt% of the bulk drug; in certain embodiments, in an amount accounting for about 50 wt% to about 100 wt% of the bulk drug; in certain embodiments, in an amount accounting for about 55 wt% to about 100 wt% of the bulk drug; in certain embodiments, in an amount accounting for about 60 wt% to about 100 wt% of the bulk drug; in certain embodiments, in an amount accounting for about 65 wt% to about 100 wt% of the bulk drug; in certain embodiments, in an amount accounting for about 70 wt% to about 100 wt% of the bulk drug; in certain embodiments, in an amount accounting for about 75 wt% to about 100 wt% of the bulk drug; in certain embodiments, in an amount accounting for about 80 wt% to about 100 wt% of the bulk drug; in certain embodiments, in an amount accounting for about 85 wt% to about 100 wt% of the bulk drug; in certain embodiments, in an amount accounting for about 90 wt% to about 100 wt% of the bulk drug; in certain embodiments, in an amount accounting for about 95 wt% to about 100 wt% of the bulk drug; in certain embodiments, in an amount accounting for about 98 wt% to about 100 wt% of the bulk drug; in certain embodiments, in an amount accounting for about 99 wt% to about 100 wt% of the bulk drug; and in certain embodiments, the crystal form B, crystal form C or crystal form D is present in an amount substantially accounting for 100 wt% of the bulk drug, that is, the bulk drug is a substantially pure crystal.

The present invention also provides a method for preparing the crystal form B, crystal form C or crystal form D, which method comprises:
(1) adding a crude free base of the compound as shown in formula I to a solvent, suspending and stirring same, and filtering the mixture to obtain the crystal form; or
(2) adding a crude free base of the compound as shown in formula I to a solvent, heating the mixture until the solution is clear, cooling the solution for crystallization, and filtering the mixture to obtain the crystal form.

In some embodiments, the crystal form B is obtained by suspending a free base of compound I in an IPAc solvent and stirring same for 2-5 days.

In some embodiments, the crystal form B is obtained by adding a free base of compound I to a mixed solvent of a nitrile solvent and an alcohol solvent, heating the mixture until the solution is clear, and then cooling the solution for crystallization.

In some embodiments, the nitrile solvent is selected from acetonitrile, and the alcohol solvent is selected from methanol, ethanol, propyl alcohol, isopropyl alcohol or a combination thereof.

In some embodiments, the solvent is a mixed solvent of acetonitrile and ethanol.

In some embodiments, the volume ratio of the nitrile solvent to the alcohol solvent is (1-3) : (1-3); in some embodiments, the volume ratio of the nitrile solvent to the alcohol solvent is (1-2) : (1-2); and in some embodiments, the volume ratio of the nitrile solvent to the alcohol solvent is 1 : 1.

In some embodiments, the crystal form is obtained by first cooling the clear solution to 50°C-60°C, adding a seed crystal (which can be prepared by other methods, such as the method described in the previous embodiment), and then continuing to cool the solution for crystallization.

In some embodiments, the crystal form C is obtained by suspending a free base of compound I in H₂O and stirring same for 2-5 days.

In some embodiments, the crystal form D is obtained by adding a free base of compound I to an alcohol solvent, heating the mixture until the solution is clear, and then slowly cooling the solution for crystallization. In some embodiments, the alcohol solvent is selected from methanol, ethanol, propyl alcohol, isopropyl alcohol or a combination thereof. In some embodiments, the solvent is methanol.

The present invention has the following beneficial effects:
The crystal form of the present invention has excellent physical and chemical properties, such as good flowability and significantly improved viscosity, which can significantly reduce the filtration time, shorten the production cycle and save the costs during the formulation preparation process of the crystal form of the present invention. Moreover, the crystal form of the present invention has good light stability, thermal stability and wet stability, which can ensure the reliability of the crystal form during storage and transportation, thereby ensuring the safety and long-term efficacy of the formulation. The crystal form does not require special packaging against light, temperature and humidity, thereby reducing the costs. The crystal form will not be degraded by light, high temperature and high humidity, and patients taking the crystal form do not need to worry about photosensitivity reactions caused by the formulation due to exposure to sunlight. The crystal form also has a relatively high solubility, which is conducive to the dissolution of the drug and improves the bioavailability.

The crystal form of the present invention also has good stability. For example, the crystal form of the present invention does not change after DVS tests (60%RH-95%RH-0%RH-95%RH or 0%RH-95%RH-0%RH) or after being placed at 25°C/60%RH and 40°C/75%RH for 1 week to 1 month. In addition, the crystal form of the present invention further has grinding stability.

Furthermore, the crystal form of the present invention has a simple and effective preparation method, and its scale-up production is easily realized. The crystal form has the advantages of good flowability, good compressibility, high bulk density, low hygroscopicity and uniform particle size distribution, and is convenient for formulation production.

The formulation prepared with the crystal form of the present invention can achieve reduced irritation and improved absorption, thereby solving the problems of metabolic rate, toxicity and safety and effectively ensuring the quality and efficacy of the formulation.

It can be understood that, as is well known in the art of thermogravimetric analysis (TGA) and differential scanning calorimetry (DSC), melting peak heights of a TGA curve and a DSC curve depend on many factors related to sample preparation and geometric shapes of instruments, and peak positions are relatively insensitive to experiment details. Therefore, in some embodiments, the crystallized compounds of the present invention have TGA and DSC patterns comprising characteristic peak positions, which have substantially the same properties as the TGA and DSC patterns provided in the drawings of the present invention, with an error tolerance of measured values within ± 5°C, which is generally required to be within ± 3°C.

It can be understood that the numerical values described and claimed in the present invention are approximate values. Changes in values may be attributed to device calibration, device errors, crystal purity, crystal size, sample size and other factors.

It can be understood that the crystal forms of the present invention are not limited to the characteristic patterns such as XRPD, DSC, TGA, DVS and adsorption isotherm curve graphs which are completely identical to those described in the drawings disclosed by the present invention, and any crystal form having a characteristic pattern which is essentially or substantially the same as those described in the drawings falls within the scope of the present invention.

The term "therapeutically effective amount" means an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

The "room temperature" refers to: 10°C-30°C.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical formulation.

The term "crystal form", "crystal" or "polymorph" refers to any solid substance that exhibits a three-dimensional order, and in contrast to an amorphous solid substance, produces a characteristic XRPD pattern with well-defined peaks.

The term "seed crystal" refers to an insoluble additive that is added in the crystallization method to form a crystal nucleus, thereby accelerating or promoting the growth of enantiomeric crystals that have the same crystal form or stereo configuration as the additive.

The term "X-ray powder diffraction pattern (XRPD pattern)" refers to an experimentally observed diffraction pattern or a parameter, data or value derived therefrom. An XRPD pattern is typically characterized by peak positions (abscissa) and/or peak intensities (ordinate).

The term "2θ" refers to the position of the peak expressed in degrees (°) and set based on the X-ray diffraction experiment and is typically the unit of the abscissa in the diffraction pattern. If the reflection beam is diffracted when the incident beam forms an angle θ with a lattice plane, the experimental setup requires that the reflection beam be recorded at an angle 2θ. It should be understood that a particular 2θ value for a particular crystal form referred to herein is intended to mean the 2θ value (expressed in degrees) measured using the X-ray diffraction experimental conditions described herein.

The term "substantially the same" or "substantially as shown in Figure XX" with respect to X-ray diffraction peaks means that representative peak positions and intensity variations are taken into account. For example, those skilled in the art will appreciate that peak positions (2θ) may have some variation, typically a variation of up to 0.1-0.2°, and the instrument used to measure the diffraction can also cause some variation. In addition, those skilled in the art will appreciate that relative peak intensity may vary due to instrument-to-instrument differences, degree of crystallinity, preferential orientation, prepared sample surfaces, and other factors known to those skilled in the art and should be considered as qualitative measurements only.

### Brief Description of the Drawings

Figure 1 shows the X-ray powder diffraction pattern of the crystal form B.
Figure 2 shows the differential scanning calorimetry curve pattern of the crystal form B.
Figure 3 shows the thermogravimetric analysis pattern of the crystal form B.
Figure 4 shows the nuclear magnetic spectrum of the crystal form B.
Figure 5 shows the variable-temperature XRPD test pattern of the crystal form B.
Figure 6 shows the X-ray powder diffraction pattern of the crystal form C.
Figure 7 shows the differential scanning calorimetry curve pattern of the crystal form C.
Figure 8 shows the thermogravimetric analysis pattern of the crystal form C.
Figure 9 shows the X-ray powder diffraction pattern of the crystal form D.
Figure 10 shows the PLM image of the crystal form B.
Figure 11 shows the PLM image of the crystal form C.
Figure 12 shows the DVS plot of the crystal form B.
Figure 13 shows the DVS plot of the crystal form C.

### Detailed Description of Embodiments

The content of the present invention is described in detail with the following examples. If a specific condition is not indicated in the examples, a conventional condition is used in an experimental method. The listed examples are intended to better illustrate the content of the present invention but should not be construed as limiting the content of the present invention. According to the above-mentioned content of the invention, those skilled in the art can make unsubstantial modifications and adjustments to the embodiments, which still fall within the protection scope of the present invention.

**Table 1 Comparison table of Chinese and English names of reagents used in experiments**

| English | Chinese | English | Chinese |
|---|---|---|---|
| EtOH | 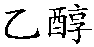 (Ethanol) | ACN | 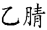 (Acetonitrile) |
| IPAc | 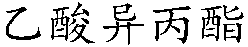 (Isopropyl acetate) | H₂O | 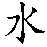 (Water) |

The XRPD measurements are analysed using an X-ray powder diffractometer, Panalytical EMPYREAN. The scanning parameters are as shown in Table 2:

**Table 2 Test parameters of XRPD**

| **Parameter** | **Instrument 1** | **Instrument 2** | **Instrument 3** |
|---|---|---|---|
| Model | Empyrean | X' Pert3 | X' Pert3 |
| X ray | Cu, Kα, Kα1 (Å): 1.540598, Kα2 (Å): 1.544426 | Cu, Kα, Kα1 (Å): 1.540598, Kα2 (Å): 1.544426 | Cu, Kα, Kα1 (Å): 1.540598, Kα2 (Å): 1.544426 |
| | Kα2/Kα1 intensity ratio: 0.50 | Kα2/Kα1 intensity ratio: 0.50 | Kα2/Kα1 intensity ratio: 0.50 |
| Settings of X-ray tube | 45 kV, 40 mA | 45 kV, 40 mA | 45 kV, 40 mA |
| Divergence slit | 1/8° | 1/8° | 1/8° |
| Scan mode | Continuous | Continuous | Continuous |
| Scan range (°2Theta) | 3-40 | 3-40 | 3-40 |
| Scanning time per step (s) | 17.8 | 46.7 | 46.7 |
| Step length of scanning (°2Theta) | 0.0167 | 0.0263 | 0.0263 |
| Test time | About 5 min 30 s | About 5 min | About 5 min |

The TGA and DSC measurements are obtained on a TA Q5000/5500 thermal gravimetric analyser and a TA 2500 differential scanning calorimeter, respectively, and the test parameters are as shown in Table 3:

**Table 3 Test parameters of DSC and TGA**

| Parameter | TGA | DSC |
|---|---|---|
| Method | Linear temperature rise | Linear temperature rise |
| Sample tray | Aluminium tray, open | Aluminium tray, capped/uncapped |
| Temperature range | Room temperature - set final temperature | 25°C - set final temperature |
| Scanning rate (°C/min) | 10 | 10 |
| Protective gas | Nitrogen gas | Nitrogen gas |

The known starting materials of the present invention can be synthesized by or according to methods known in the art, or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd. and other companies.

Unless otherwise specified in the examples, a solution refers to an aqueous solution.

### Example 1 Preparation of compound I

### (S)-N-((S)-1-cyano-2-(2-fluoro-4-(3-methyl-2-oxo-2,3-dihydrobenzo[d]oxazol-5-yl)phenyl)ethyl)-1,4-oxazepane-2-carboxamide (compound I)

### Step 1:

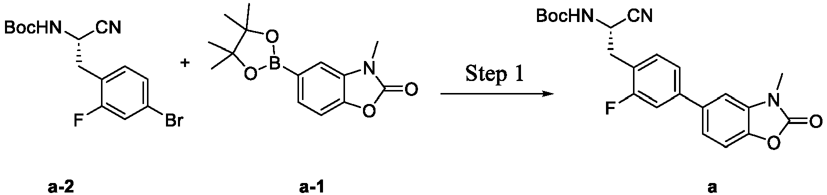

Reaction: to a 50-L reaction kettle, 1,4-dioxane (10.005 kg), compound a-1 (1.600 kg, with reference to the method in patent WO 2015110826A1) and compound a-2 (2.000 kg, with reference to the method in patent WO 2016139355A1) were added under stirring, followed by aqueous potassium carbonate solution (6.605 kg, which was prepared by dissolving potassium carbonate (1.600 kg) in purified water (5.005 kg)). After the addition, the mixture was subjected to nitrogen replacement three times. [1,1'-bis(diphenylphosphino)ferrocene]palladium dichloride dichloromethane complex (100.0 g) was added, and the mixture was subjected to nitrogen replacement once. Under nitrogen protection, the reaction liquid was warmed to 80°C ± 5°C and reacted for about 2 hours. Then, a sample was collected and monitored with HPLC, and when the target value for in-process control, i.e., the content of compound a-2 was ≤ 1.0%, the reaction was terminated.

Post treatment: to the reaction liquid was added purified water (5.000 kg), and the mixture was cooled to 10°C ± 5°C. Purified water (10.005 kg) was added, and the resulting mixture was stirred and crystallized for about 1 hour at 10°C ± 5°C and filtered. The filter cake was washed twice with purified water (2.500 kg x 2) and collected. Anhydrous ethanol (12.605 kg) and the filter cake were added to a 50-L reaction kettle, the mixture was stirred at 20°C ± 5°C for about 0.5 hours and filtered, and the filter cake was washed twice with anhydrous ethanol (1.000 kg x 2) and collected.

Drying: the filter cake was dried at 55°C ± 5°C under vacuum of ≤ -0.07 MPa for about 16 hours and collected to obtain compound a (2.143 kg, molar yield: 89.4%).

¹H NMR (400 MHz, DMSO) δ 7.90 (s, 1H), 7.72 - 7.30 (m, 6H), 4.72 (s, 1H), 3.41 (d, 3H), 3.09-3.21(m, 2H), 1.37 (s, 9H).

LCMS m/z =356.1[M-56+H]⁺.

### Step 2:

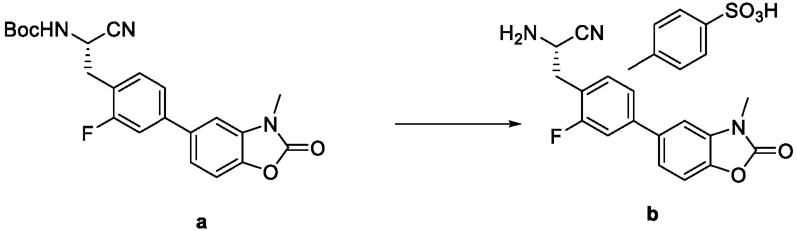

Reaction: to a 50-L glass reaction kettle, acetonitrile (16.785 kg), compound a (2.1381 kg) and p-toluenesulphonic acid monohydrate (2.950 kg) were added under stirring. After the addition, the temperature was controlled at 25°C ± 5°C and the mixture was reacted for about 2 hours. Then, a sample was collected and monitored with HPLC, and when the target value for in-process control, i.e., the content of compound a was ≤ 1.0%, the reaction was terminated.

Post treatment: the reaction mixture was filtered, and the filter cake was washed with acetonitrile (1.670 kg) and collected. Acetonitrile (2.1381 kg) and filter cake were added to a reaction kettle, and the mixture was warmed to 80°C ± 5°C and stirred for 3 hours. The resulting mixture was cooled to 20°C ± 5°C and stirred for 1 hour. The reaction mixture was filtered, and the filter cake was washed with acetonitrile (1.670 kg) and collected.

Drying: the filter cake was dried at 55°C ± 5°C under vacuum of ≤ -0.07 MPa for about 16 hours and collected to obtain compound b (2.141 kg, yield: 85.5%).

¹H NMR (400 MHz, DMSO) δ 9.04 (s, 3H), 7.70 - 7.37 (m, 8H), 7.13 (d, 2H), 4.90 (dd, 1H), 3.41 (s, 3H), 3.29 (t, 2H), 2.29 (s, 3H).

LCMS m/z =312.2 [M-172+H]⁺.

### Step 3:

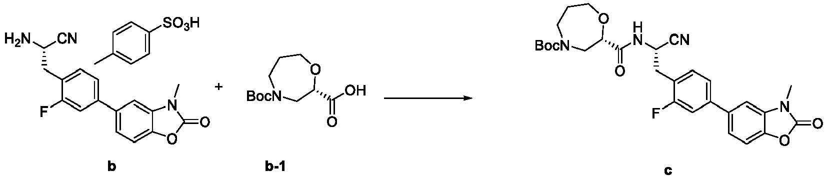

Reaction: to a 100-L reaction kettle, ethyl acetate (19.060 kg), compound b (2.1413 kg) and compound b-1 (1.1300 kg, which was purchased from Pharmablock Sciences (Nanjing), Inc.) were added under stirring, followed by N,N-diisopropylethylamine (1.725 kg). After the addition, under nitrogen protection, the reaction liquid was cooled to 5°C ± 5°C, and propylphosphonic anhydride (4.240 kg) was added dropwise while the temperature was controlled at 10°C ± 5°C. After the addition, the temperature was maintained at 25°C ± 5°C and the mixture was reacted for about 3-8 hours.

Post treatment: the reaction liquid was washed successively with sodium bicarbonate solution (which was prepared by dissolving 1.070 kg of sodium bicarbonate in 20.350 kg of water), citric acid solution (which was prepared by dissolving 2.150 kg of citric acid monohydrate in 19.275 kg of water) and sodium chloride (which was prepared by dissolving 4.300 kg of sodium chloride in 17.135 kg of water). To the organic phase was added medicinal charcoal (0.210 kg), and the mixture was stirred for about 0.5 hours. The resulting mixture was filtered through a pad of celite (0.540 kg), and the filter cake was washed with ethyl acetate (1.905 kg). The organic phase was dried for about 0.5 hours by adding anhydrous sodium sulphate (1.070 kg). The mixture was filtered, the filter cake was washed with ethyl acetate (1.905 kg), and the filtrates were combined.

Concentration: the resulting filtrate was concentrated under reduced pressure at 50°C ± 5°C until no obvious fraction flew out, to obtain compound c (2.385 kg, overweight, and the amount was calculated based on a 100% yield). The compound was directly used for the next reaction.

¹H NMR (400 MHz, CDCl₃) δ 7.54 - 7.01 (m, 7H), 5.18 (s, 1H), 4.25 - 3.94 (m, 3H), 3.54 (dd, 2H), 3.46 (s, 3H), 3.39 - 3.04 (m, 4H), 1.99 (d, 2H), 1.54 - 1.39 (m, 9H).

LCMS m/z =483.2 [M-56+1]⁺.

### Step 4:

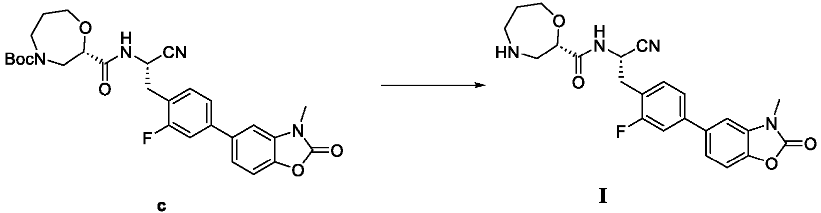

Reaction: to a 50-L double-layer glass reaction kettle containing the concentrate of compound c, acetonitrile (9.305 kg) and p-toluenesulphonic acid monohydrate (2.530 kg) were added under stirring. After the addition, the temperature was maintained at 25°C ± 5°C and the mixture was reacted for about 2 hours. Then, a sample was collected and monitored with HPLC, and when the target value for in-process control, i.e., the content of compound c was ≤ 1.0%, the reaction was terminated.

Post treatment: the reaction liquid was cooled to 10°C ± 5°C, and dilute aqueous ammonia (a mixture of 1.075 kg of aqueous ammonia and 36.000 kg of purified water) was added dropwise while the material temperature was controlled below 25°C. After the addition, the mixture was cooled to 10°C ± 5°C to allow crystallization for 2 hours. The resulting mixture was filtered, and the filter cake was washed with purified water (11.930 kg). The filter cake and ethanol (14.890 kg) were added to a 50-L double-layer glass reaction kettle and the mixture was stirred at 20°C ± 5°C for 0.5 hours and filtered. The filter cake was washed with ethanol (1.860 kg) and collected. The filter cake was dried at 55°C ± 5°C under vacuum (vacuum degree ≤ -0.07 MPa) for about 13 hours and collected to obtain crude compound I (1.6627 kg, molar yield: 85.6%).

Refining: to a 100-L reaction kettle were added acetonitrile (9.100 kg), anhydrous ethanol (9.220 kg) and crude compound I (1.6627 kg) under stirring, and the mixture was heated to an internal temperature of 75°C ± 5°C, stirred until a clear solution was obtained, and filtered while hot. The filtrate was transferred into a 100-L reaction kettle (if a product was precipitated out from the filtrate, the filtrate should be heated until the solution was clear) and cooled to 35°C ± 5°C under stirring. The temperature was maintained constant until a solid was precipitated out and then the mixture was stirred for about 0.5 hours while the temperature was maintained constant. The resulting mixture was then cooled to 5°C ± 5°C, and the temperature was maintained constant to allow crystallization for 2 hours. The mixture was filtered, and the filter cake was washed with ethanol (1.300 kg) and collected.

Drying: the filter cake was dried at 55°C ± 5°C under vacuum (vacuum degree ≤ -0.07 MPa) for about 25 hours to obtain the free base of the compound as shown in formula I (1.4060 kg, molar yield: 84.6%).

¹H NMR (400 MHz, DMSO) δ 8.69 (d, 1H), 7.64 (d, 1H), 7.61 - 7.51 (m, 2H), 7.46 (t, 2H), 7.39 (d, 1H), 5.06 (q, 1H), 4.01 (dd, 1H), 3.87 (ddd, 1H), 3.73 (ddd, 1H), 3.40 (s, 3H), 3.34 - 3.28 (m, 1H), 3.20 (dd, 1H), 3.06 (dd, 1H), 2.78 (ddd, 1H), 2.69 - 2.54 (m, 2H), 2.21 (s, 1H), 1.84 - 1.63 (m, 2H).

LCMS m/z =439.2 [M+1]⁺.

### Example 2 Preparation of crystal form B of compound I

The compound (200 mg) prepared in Example 1 was weighed, suspended in an IPAc solvent at room temperature and stirred for 3 days and the mixture was filtered and dried. The sample was tested by XRPD and the free base crystal form was described as crystal form B.

The XRPD pattern thereof is as shown in Figure 1, and the XRPD diffraction peaks are listed in Table 4.

**Table 4**

| Pos. [°2θ] | Height [cts] | FWHM Left [°2θ] | d-spacing [Å] | Rel. Int. [%] |
|---|---|---|---|---|
| 4.68 | 33.71 | 0.3070 | 18.99 | 4.87 |
| 8.97 | 60.17 | 0.3070 | 9.88 | 8.69 |
| 12.32 | 427.57 | 0.1023 | 7.20 | 61.76 |
| 14.30 | 591.05 | 0.1023 | 6.21 | 85.38 |
| 15.43 | 692.25 | 0.1023 | 5.75 | 100.00 |
| 16.38 | 393.10 | 0.1023 | 5.42 | 56.79 |
| 17.06 | 193.58 | 0.1023 | 5.21 | 27.96 |
| 18.21 | 196.03 | 0.0768 | 4.88 | 28.32 |
| 18.56 | 328.17 | 0.1279 | 4.79 | 47.41 |
| 18.94 | 127.20 | 0.1535 | 4.69 | 18.37 |
| 19.59 | 174.31 | 0.1279 | 4.54 | 25.18 |
| 20.79 | 106.65 | 0.2047 | 4.28 | 15.41 |
| 21.19 | 95.77 | 0.1535 | 4.20 | 13.84 |
| 22.55 | 72.39 | 0.1535 | 3.95 | 10.46 |
| 22.97 | 75.26 | 0.1535 | 3.88 | 10.87 |
| 23.37 | 88.23 | 0.1535 | 3.81 | 12.75 |
| 24.25 | 253.56 | 0.1279 | 3.67 | 36.63 |
| 25.46 | 97.34 | 0.1535 | 3.50 | 14.06 |
| 31.03 | 49.81 | 0.3070 | 2.88 | 7.20 |

The DSC/TGA results are shown in Figures 2 and 3 and showed that the sample had a weight loss of 2.3% when heated to 150°C and had one endothermic peak at 181.9°C (initial temperature). The nuclear magnetic resonance results (Figure 4) showed that the molar ratio of residual solvent IPAc to API in the sample was 0.01 (corresponding to a weight loss of 0.1 wt%). In order to study the TGA weight loss of the crystal form B, a variable-temperature XRPD test was performed. The results (Figure 5) showed that the free base crystal form B did not change when the crystal form was purged with nitrogen for 20 minutes, heated to 120°C, or cooled to 30°C (some diffraction peaks shifted at 120°C, and it is speculated that the shift may be caused by lattice expansion at high temperature). Therefore, it is speculated that the free base crystal form B is an anhydrous crystal form.

### Example 3 Preparation of crystal form B of compound I

The compound (1.0 g) prepared in Example 1 was weighed, and acetonitrile (5 ml) and ethanol (5 ml) were added. The mixture was warmed until a clear solution was obtained, and then slowly cooled to 50°C-60°C, a seed crystal (Example 2) was added, and the resulting mixture was cooled to room temperature, filtered and dried. The sample was characterized by XRPD, DSC and TGA and was the crystal form B.

### Example 4 Preparation of crystal form B of compound I

The compound (4.0 g) prepared in Example 1 was weighed, and dimethyl sulphoxide (15 ml) was added. The mixture was warmed to 75°C until a clear solution was obtained. After a clear solution was obtained, ethanol (70 ml) was added, and the resulting mixture was stirred for 30 min while the temperature was maintained constant. The seed crystal obtained in Example 3 was added and the mixture was cooled to room temperature, filtered and dried to obtain the crystal form B (3.36 g, yield: 84.0%).

### Example 5 Preparation of monohydrate crystal form C of compound I

The compound (200 mg) prepared in Example 1 was weighed, suspended in H₂O at room temperature and stirred for 3 days and the mixture was filtered and dried. The sample was tested by XRPD and the crystal form was described as crystal form C.

The XRPD pattern thereof is as shown in Figure 6, and the XRPD diffraction peaks are listed in Table 5.

**Table 5**

| Pos. [°2θ] | Height [cts] | FWHM Left [°2θ] | d-spacing [Å] | Rel. Int. [%] |
|---|---|---|---|---|
| 4.68 | 219.33 | 0.1023 | 18.73 | 9.63 |
| 8.93 | 1290.12 | 0.1279 | 9.91 | 56.63 |
| 12.10 | 2277.96 | 0.1279 | 7.32 | 100.00 |
| 14.14 | 736.84 | 0.1023 | 6.26 | 32.35 |
| 15.19 | 380.97 | 0.1023 | 5.83 | 16.72 |
| 15.92 | 611.99 | 0.0768 | 5.57 | 26.87 |
| 16.06 | 571.05 | 0.0768 | 5.52 | 25.07 |
| 17.55 | 400.98 | 0.1279 | 5.06 | 17.60 |
| 17.90 | 269.48 | 0.1023 | 4.96 | 11.83 |
| 18.90 | 418.70 | 0.1023 | 4.70 | 18.38 |
| 19.10 | 517.76 | 0.1279 | 4.65 | 22.73 |
| 20.38 | 1834.01 | 0.1535 | 4.36 | 80.51 |
| 20.81 | 296.51 | 0.0768 | 4.27 | 13.02 |
| 23.32 | 159.67 | 0.1535 | 3.81 | 7.01 |
| 23.86 | 882.25 | 0.1023 | 3.73 | 38.73 |
| 24.33 | 262.17 | 0.1023 | 3.66 | 11.51 |
| 25.07 | 1679.46 | 0.1279 | 3.55 | 73.73 |
| 25.91 | 202.59 | 0.1023 | 3.44 | 8.89 |
| 26.99 | 160.46 | 0.1279 | 3.31 | 7.04 |
| 28.45 | 199.66 | 0.1535 | 3.14 | 8.76 |
| 29.67 | 46.66 | 0.5117 | 3.01 | 2.05 |
| 31.41 | 105.45 | 0.1535 | 2.85 | 4.63 |
| 36.26 | 124.59 | 0.1791 | 2.48 | 5.47 |

The DSC/TGA results are shown in Figures 7 and 8, and the results showed that the sample had a weight loss of 4.5% when heated to 150°C and had three endothermic peaks at 103.5°C, 113.4°C and 183.0°C (peak temperatures). The sample was heated to 120°C and cooled to room temperature, and then an XRPD test was performed. The results showed that the crystal form C was transformed into the crystal form B upon heating. Based on the fact of crystal transformation of the sample upon heating in combination with the stepwise weight loss of the TGA, it is speculated that the crystal form C is a monohydrate.

### Example 6 Preparation of crystal form D of compound I

The compound (200 mg) prepared in Example 1 was weighed and added to methanol (20 mg). The mixture was heated to reflux and filtered while hot. The resulting mixture was slowly cooled to room temperature for crystallization, filtered and dried. The sample was characterized by XRPD and the crystal form was described as crystal form D.

The XRPD pattern thereof is as shown in Figure 9, and the XRPD diffraction peaks are listed in Table 6.

**Table 6**

| Pos. [°2θ] | Height [cts] | FWHM Left [°2θ] | d-spacing [Å] | Rel. Int. [%] |
|---|---|---|---|---|
| 4.49 | 144.92 | 0.0502 | 19.66 | 8.96 |
| 8.68 | 1524.04 | 0.0502 | 10.19 | 94.18 |
| 8.99 | 238.85 | 0.0502 | 9.84 | 14.76 |
| 11.74 | 480.36 | 0.0502 | 7.54 | 29.69 |
| 13.51 | 250.83 | 0.0502 | 6.56 | 15.50 |
| 13.64 | 1618.17 | 0.0502 | 6.49 | 100.00 |
| 14.80 | 54.46 | 0.1004 | 5.99 | 3.37 |
| 15.59 | 91.61 | 0.0669 | 5.68 | 5.66 |
| 17.40 | 729.45 | 0.0836 | 5.10 | 45.08 |
| 18.23 | 392.36 | 0.0669 | 4.87 | 24.25 |
| 20.12 | 408.80 | 0.0669 | 4.41 | 25.26 |
| 22.82 | 54.82 | 0.1004 | 3.90 | 3.39 |
| 23.60 | 102.97 | 0.0836 | 3.77 | 6.36 |
| 24.08 | 100.02 | 0.1338 | 3.70 | 6.18 |
| 26.21 | 397.75 | 0.0502 | 3.40 | 24.58 |
| 27.49 | 221.83 | 0.0502 | 3.24 | 13.71 |
| 28.57 | 45.61 | 0.1004 | 3.12 | 2.82 |
| 32.19 | 335.63 | 0.0502 | 2.78 | 20.74 |
| 32.97 | 275.04 | 0.0502 | 2.72 | 17.00 |
| 36.98 | 28.34 | 0.1673 | 2.43 | 1.75 |

### Crystal form evaluation experiments

### 1. Crystal form transformation

In order to study the transformation relationship between hydrate crystal form C and anhydrous crystal form B, a suspension competition experiment was carried out. A certain amount of free base was weighed, the corresponding solvent was added, and the mixture was suspended and stirred at room temperature for about 2 hours. Then, the resulting mixture was filtered through a 0.45 µm PTFE filter membrane to obtain a saturated solution. A sample of the free base crystal form B and crystal form C (5-7 mg) was weighed and added to a HPLC vial, the saturated solution (1.0 mL) was added, and the mixture was suspended and stirred at room temperature. Then, the solid was tested by XRPD. The results are summarized in Table 7 and showed that the free base crystal form B was obtained in a system with a water activity of 0-0.2, and the crystal form C was obtained in a system with a water activity of 0.4-1.

**Table 7**

| Initial crystal form | Solvent (v/v) | Solvent (v/v) | Temperature | Time | Results |
|---|---|---|---|---|---|
| Crystal forms B+C | EtOH | EtOH (aw of about 0) | Room temperature | 2 days | Crystal form B |
| | EtOH/H2O | EtOH/H2O (97 : 3, aw of about 0.2) | Room temperature | 2 days | Crystal form B |
| | EtOH/H2O | EtOH/H2O (93 : 7, aw of about 0.4) | Room temperature | 2 days | Crystal form C |
| | EtOH/H2O | EtOH/H2O (86 : 14, aw of about 0.6) | Room temperature | 2 days | Crystal form C |
| | EtOH/H2O | EtOH/H2O (70 : 30, aw of about 0.8) | Room temperature | 2 days | Crystal form C |
| | H2O | H2O (aw of about 1.0) | Room temperature | 2 days | Crystal form C |

### 2. PLM

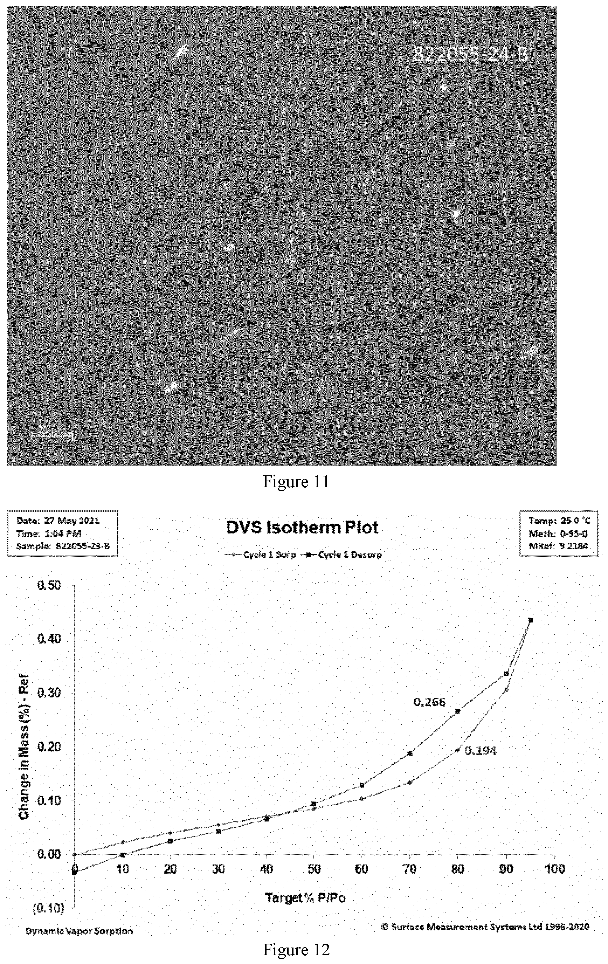

PLM characterization was performed on the crystal form B and crystal form C, and the results (Figures 10 and 11) showed that the particle size of each sample was less than 20 µm.

### 3. Hygroscopicity

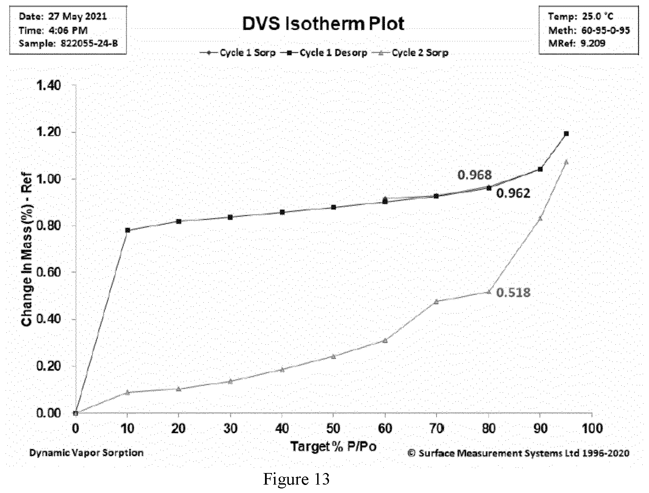

The hygroscopicity of the crystal forms B and C was evaluated by a dynamic vapour sorption (DVS) instrument. The test collected the percentage change in the mass of the samples when the humidity changed (60%RH-95%RH-0%RH-95%RH or 0%RH-95%RH-0%RH) at a constant temperature of 25°C, wherein for the crystal form C, the test started at ambient humidity (about 60%RH), and for the crystal form B, the test started at 0% relative humidity (0%RH). The test results are shown in Figures 12 and 13, respectively. The results showed that the moisture sorption of the crystal form B and crystal form C at 25°C/80%RH were 0.19% and 0.97%, respectively. The crystal form of all samples did not change after the DVS test. This indicates that the crystal form of the present invention has low hygroscopicity and low requirements for drug packaging and storage conditions.

### 4. Solid stability

For the compounds used in this experiment, the crystal form B was obtained from Example 3, and the crystal form C was obtained from Example 5

The crystal forms B and C were placed at 25°C/60%RH and 40°C/75%RH without any protection, and then the physical and chemical stabilities thereof were tested by XRPD and UPLC, respectively. The results are summarized in Table 8. The results showed that after the samples were placed at 25°C/60%RH and 40°C/75%RH for 1 week to 1 month, the crystal form of each sample did not change, and the purity thereof did not decrease significantly, indicating good stability.

**Table 8**

| Initial sample | Condition | Time point | Purity | Crystal form change |
|---|---|---|---|---|
| Crystal form B | Initial value | - | 99.86 | - |
| | 25°C/60%RH | 1 week | 99.74 | No |
| | | 3 weeks | 99.74 | N/A |
| | | 1 month | 99.76 | No |
| | 40°C/75%RH | 1 week | 99.65 | No |
| | | 3 weeks | 99.57 | N/A |
| | | 1 month | 99.63 | No |
| Crystal form C | Initial value | - | 99.87 | - |
| | 25°C/60%RH | 1 week | 99.70 | No |
| | 40°C/75%RH | 1 week | 99.71 | No |

### 5. Stability data

The compounds used in this experiment were obtained from Example 4.

### 5.1 Accelerated experiment

Examination conditions: 40°C ± 2°C, 75%RH ± 5% RH.

Packaging: the inner packaging material is a double-layer medicinal low-density polyethylene bag, and the outer packaging material is a polyester/aluminium/polyethylene medicinal composite bag.

**Table 9 Results of accelerated experiment**

| Examination item | Time point (month) | | | | |
|---|---|---|---|---|---|
| | 0 | 1 | 2 | 3 | 6 |
| Crystal form | Crystal form B | Crystal form B | Crystal form B | Crystal form B | Crystal form B |
| Moisture | 0.2% | 0.3% | 0.2% | 0.2% | 0.2% |
| Purity | 99.93% | 99.87% | 99.88% | 99.87% | 99.88 % |
| Content | 100.5% | 100.4% | 101.1% | 100.1% | 101.3% |

### 5.2 Long-term experiment 1

Examination conditions: 25°C ± 2°C, 60%RH ± 5% RH.

Packaging: the inner packaging material is a double-layer medicinal low-density polyethylene bag, and the outer packaging material is a polyester/aluminium/polyethylene medicinal composite bag.

**Table 10 Results of long-term experiment 1**

| **Examination item** | **Time point (month)** | | | | |
|---|---|---|---|---|---|
| | **0** | **3** | **6** | **9** | **12** |
| **Crystal form** | Crystal form B | Crystal form B | Crystal form B | Crystal form B | Crystal form B |
| **Moisture** | 0.2% | 0.3% | 0.2% | 0.1% | 0.2% |
| **Purity** | 99.93% | 99.87% | 99.90% | 99.89% | 99.88% |
| **Content** | 100.5% | 100.6% | 100.7% | 101.6% | 99.5% |

### 5.3 Long-term experiment 2

Examination conditions: 30°C ± 2°C, 65%RH ± 5%RH.

Packaging: the inner packaging material is a double-layer medicinal low-density polyethylene bag, and the outer packaging material is a polyester/aluminium/polyethylene medicinal composite bag.

**Table 11 Results of long-term experiment 2**

| **Examination item** | **Time point (month)** | | | | |
|---|---|---|---|---|---|
| | **0** | **3** | **6** | **9** | **12** |
| **Crystal form** | Crystal form B | Crystal form B | Crystal form B | Crystal form B | Crystal form B |
| **Moisture** | 0.2% | 0.3% | 0.2% | 0.1% | 0.2% |
| **Purity** | 99.93% | 99.87% | 99.90% | 99.90% | 99.89% |
| **Content** | 100.5% | 100.6% | 101.6% | 100.7% | 99.2% |

### 5.4 Long-term experiment 3

Examination conditions: 2°C-8°C.

Packaging: the inner packaging material is a double-layer medicinal low-density polyethylene bag, and the outer packaging material is a polyester/aluminium/polyethylene medicinal composite bag.

**Table 12 Results of long-term experiment 3**

| **Examination item** | **Time point (month)** | | | | |
|---|---|---|---|---|---|
| | **0** | **3** | **6** | **9** | **12** |
| **Crystal form** | Crystal form B | Crystal form B | Crystal form B | Crystal form B | Crystal form B |
| **Moisture** | 0.2% | 0.3% | 0.3% | 0.2% | 0.2% |
| **Purity** | 99.93% | 99.87% | 99.89% | 99.89% | 99.89% |
| **Content** | 100.5% | 100.5 % | 100.0% | 101.6% | 101.0% |

In particular, the analysis method of purity is as follows:

| **High performance liquid chromatograph (HPLC)** | | | | |
|---|---|---|---|---|
| Instrument | Model | HPLC 1260infinity | | |
| Parameter and method | Chromatographic column | Hypersil GOLD^{™} C18, 4.6 mm × 250 mm, 5 µm | | |
| | Trap column | Ghost-Sniper Column 4.6 mm × 50 mm | | |
| | Column temperature | 30°C | | |
| | Flow rate | 1.0 mL/min | | |
| | Detection wavelength | 226 nm | | |
| | Injection volume | 10 µL | | |
| | Run time | 31 min | | |
| | Mobile phase | mobile phase A: 0.02 mol/L ammonium dihydrogen phosphate solution (adjusted to pH 4.0 with phosphoric acid); mobile phase B: methanol : acetonitrile (50 : 50) | | |
| | Running gradient | Time (min) | A (%) | B (%) |
| | | 0 | 60 | 40 |
| | | 5 | 60 | 40 |
| | | 10 | 40 | 60 |
| | | 20 | 20 | 80 |
| | | 25 | 20 | 80 |
| | | 26 | 60 | 40 |
| | | 31 | 60 | 40 |

### Bioexperiments

### 1. In vitro DPP1 enzyme activity assay

Recombinant human DPP1 enzyme (R&D Systems, Cat. No. 1071-CY) at a final concentration of 100 µg/mL was mixed with recombinant human cathepsin L (R&D Systems, Cat. No. 952-CY) at a final concentration of 20 µg/mL were mixed and incubated at room temperature for 1 hour to activate the DPP1 enzyme. The activated DPP1 enzyme was diluted 100-fold, and 5 µL of compounds at different concentrations and 5 µL of the diluted DPP1 enzyme were added to a 384-well plate and incubated at room temperature for 30 minutes. After 10 µL of the substrate Gly-Arg-AMC (bachem, Cat. No. I-1215) at a concentration of 20 µM was added, incubation was continued at room temperature for 60 minutes, and the fluorescence intensity was detected with a microplate reader (excitation light = 380 nm and emission light = 460 nm). IC₅₀ values were calculated using the DosResp function of the Origin2019 software.

**Table 13 DPP1 inhibitory activity**

| Compound No. | IC₅₀/nM |
|---|---|
| Compound I | 1.6 |

Conclusion: compound I showed relatively high inhibitory activity against the DPP1 receptor.

### 2. Pharmacokinetic test in rats

1.1 Experimental animals: male SD rats, about 220 g, 6-8 weeks old, 6 rats/compound. Purchased from Chengdu Ddossy Experimental Animals Co., Ltd.

1.2 Experimental design: on the day of the experiment, 6 SD rats were randomly grouped according to their body weights. The animals were fasted with water available for 12 to 14 h one day before the administration and were fed 4 h after the administration.

**Table 14 Administration information**

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of administration |
| G1 | 3 | INS1007 | 1 | 0.2 | 5 | Plasma | Intravenous administration |
| G2 | 3 | Compound I | 1 | 0.2 | 5 | Plasma | |
| G3 | 3 | INS1007 | 3 | 0.3 | 10 | Plasma | Intragastric administration |
| G4 | 3 | Compound I | 3 | 0.3 | 10 | Plasma | |

Vehicle for intravenous administration: 5% DMA+5% Solutol+90% Saline; vehicle for intragastric administration: 0.5% MC; control compound INS1007, namely compound 2 in patent WO 2015110826A1, was prepared with reference to the method of the patent.

Before and after the administration, 0.1 ml of blood was taken from the orbit of the animals under isoflurane anaesthesia and placed in an EDTAK2 centrifuge tube. The blood was centrifuged at 5000 rpm and 4°C for 10 min to collect the plasma. Blood sampling time points for the intravenous administration group: 0, 5 min, 15 min, 30 min, 1 h, 2 h, 4 h, 6 h, 8 h, and 24 h; and blood sampling time points for the intragastric administration group: 0, 5 min, 15 min, 30 min, 1 h, 2 h, 4 h, 6 h, 8 h, and 24 h. Before analysis and detection, all samples were stored at - 80°C.

**Table 15 Pharmacokinetic parameters of test compounds in plasma of rats**

| Test compounds | Mode of administration | CL (mL/min/kg) | Vdₛₛ (L/kg) | AUC₀₋ₜ (hr*ng/mL) | F (%) |
|---|---|---|---|---|---|
| INS 1007 | Intravenous administration (1 mg/kg) | 2.08 | 0.738 | 8396 | - |
| Compound I | | 1.66 | 0.753 | 9503 | - |
| INS 1007 | Intragastric administration (3 mg/kg) | - | - | 22201 | 88.1 |
| Compound I | | - | - | 31159 | > 100 |

Conclusion: the compound of the present invention has relatively good bioavailability and pharmacokinetic characteristics.

### 3. Toxicity experiment in rats following 14-day repeated oral administration

According to their body weight, the SD rats were randomly divided into the following groups: vehicle control group (0.5% MC), INS1007 (30, 100, and 300 mg/kg) groups, and compound I (30, 100, and 300 mg/kg) groups. For the administration groups, 16 rats were included in each group, and for the vehicle control group, 10 rats were included, with an equal number of male and female rats in each of the groups. The rats were orally gavaged with the drug or vehicle at the corresponding concentrations every day for 14 consecutive days, with a recovery period of 7 days. During the administration period, general symptoms were observed, and body weight and food intake were measured for each group. At the end of the administration period and at the end of the recovery period, haematology tests, serum biochemistry test and gross anatomy were performed on the rats in each group separately.

Conclusion: the compound of the present invention is less toxic than INS 1007 and thus is safer at the same dosage.

## Claims

1. A polymorph of a compound as shown in formula I: 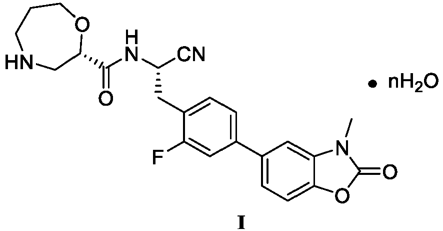 **characterised in that** n is 0 or 1.

2. The polymorph according to claim 1, **characterised in that** n is 0, and the polymorph is crystal form B, which has an X-ray powder diffraction pattern comprising characteristic diffraction peaks at 12.32 ± 0.2°, 14.30 ± 0.2°, 15.43 ± 0.2°, 16.38 ± 0.2° and 18.56 ± 0.2° 2θ, as determined by using Cu-Kα radiation.

3. The polymorph according to claim 2, **characterised in that** the polymorph has an X-ray powder diffraction pattern further comprising characteristic diffraction peaks at 17.06 ± 0.2°, 18.21 ± 0.2°, 18.94 ± 0.2°, 19.59 ± 0.2°, 20.79 ± 0.2°, 21.19 ± 0.2°, 22.55 ± 0.2°, 22.97 ± 0.2°, 23.37 ± 0.2°, 24.25 ± 0.2° and 25.46 ± 0.2° 2θ.

4. The polymorph according to claim 2 or 3, **characterised in that** the polymorph has an X-ray powder diffraction pattern substantially as shown in Figure 1.

5. The polymorph according to claim 2 or 3, **characterised in that** the crystal form B has a DSC pattern as shown in Figure 2 and/or a TGA pattern as shown in Figure 3.

6. The polymorph according to claim 1, **characterised in that** n is 1, and the polymorph is crystal form C, which has an X-ray powder diffraction pattern comprising characteristic diffraction peaks at 8.93 ± 0.2°, 12.10 ± 0.2°, 14.14 ± 0.2°, 20.38 ± 0.2°, 23.86 ± 0.2° and 25.07 ± 0.2° 2θ, as determined by using Cu-Kα radiation.

7. The polymorph according to claim 6, **characterised in that** the polymorph has an X-ray powder diffraction pattern further comprising characteristic diffraction peaks at 15.19 ± 0.2°, 15.92 ± 0.2°, 16.06 ± 0.2°, 17.55 ± 0.2°, 17.90 ± 0.2°, 18.90 ± 0.2°, 19.10 ± 0.2°, 20.81 ± 0.2° and 24.33 ± 0.2° 2θ.

8. The polymorph according to claim 6 or 7, **characterised in that** the polymorph has an X-ray powder diffraction pattern substantially as shown in Figure 6.

9. The polymorph according to claim 6 or 7, **characterised in that** the crystal form C has a DSC pattern as shown in Figure 7 and/or a TGA pattern as shown in Figure 8.

10. The polymorph according to claim 1, **characterised in that** n is 0, and the polymorph is crystal form D, which has an X-ray powder diffraction pattern comprising characteristic diffraction peaks at 8.68 ± 0.2°, 11.74 ± 0.2°, 13.64 ± 0.2°, 17.40 ± 0.2°, 18.23 ± 0.2°, 20.12 ± 0.2° and 26.21 ± 0.2° 2θ, as determined by using Cu-Kα radiation.

11. The polymorph according to claim 10, **characterised in that** the polymorph has an X-ray powder diffraction pattern further comprising characteristic diffraction peaks at 8.99 ± 0.2°, 13.51 ± 0.2°, 27.49 ± 0.2°, 32.19 ± 0.2° and 32.97 ± 0.2° 2θ.

12. The polymorph according to claim 10 or 11, **characterised in that** the polymorph has an X-ray powder diffraction pattern substantially as shown in Figure 9.

13. A pharmaceutical composition, **characterised in that** the pharmaceutical composition comprises a therapeutically effective amount of the polymorph according to any one of claims 1-12, and a pharmaceutically acceptable carrier and/or excipient.

14. Use of the polymorph according to any one of claims 1-12 or the pharmaceutical composition according to claim 13 in the preparation of a medicament for treating a DPP1-mediated disease.

15. The use according to claim 14, **characterised in that** the DPP1-mediated disease is selected from non-cystic fibrosis bronchiectasis, cystic fibrosis bronchiectasis, acute lung injury, obstructive airway disease, bronchiectasis, cystic fibrosis, asthma, emphysema and chronic obstructive pulmonary disease.
